# EUROPÄISCHE PATENTSCHRIFT

(11) **EP 0 579 908 B1**
(45) Veröffentlichungstag und Bekanntmachung des Hinweises auf die Patenterteilung: **10.09.1997**
(21) Anmeldenummer: 93106887.8
(22) Anmeldetag: 28.04.1993
(51) Int. Cl.: C07D 213/74, C07F 7/08, A01N 43/40, A01N 55/00

(54) **Pyridyl-Derivate, Verfahren zu ihrer Herstellung und ihre Verwendung als Schädlingsbekämpfungsmittel**
Pyridyl derivatives, process for their preparation and their use as pesticides
Dérivés de pyridyle, procédé pour leur préparation et leur utilisation comme pesticide

(30) Priorität: 11.05.1992 DE 4215469
(43) Veröffentlichungstag der Anmeldung: 26.01.1994
(73) Patentinhaber: BAYER AG, 51368 Leverkusen (DE)
(72) Erfinder: Knüppel, Peter C., Dr., W-5632 Wermelskirchen 3 (DE); Klausener, Alexander, Dr., W-5000 Köln 1 (DE); Tiemann, Ralf, Dr., W-5090 Leverkusen 1 (DE); Dutzmann, Stefan, Dr., W-4010 Hilden 1 (DE); Dehne, Heinz-Wilhelm, Dr., W-4019 Monheim (DE); Wachendorff-Neumann, Ulrike, Dr., W-4019 Monheim (DE)

(56) Entgegenhaltungen:
- EP-A- 0 212 859
- EP-A- 0 383 117
- GB-A- 2 238 308

## Beschreibung

Die Erfindung betrifft neue Pyridyl-Derivate, mehrere Verfahren zu ihrer Herstellung und ihre Verwendung als Schädlingsbekämpfungsmittel.

Es ist bekannt, daß bestimmte pyridylsubstituierte Acrylsäureester wie beispielsweise die Verbindung 3-Methoxy-1-{N-[6-(4-chlorphenyl-ethinyl)-2-pyridyl]-N-methyl}-amino-acrylsäuremethylester oder die Verbindung 3-Methoxy-1-{N-[6-(phenyl-ethinyl)-2-pyridyl]-N-methyl}-amino-acrylsäuremethylester fungizide Eigenschaften besitzen (vergleiche z. B. EP 383 117 US-5120734, GB-2238308).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue Pyridyl-Derivate der allgemeinen Formel (I), 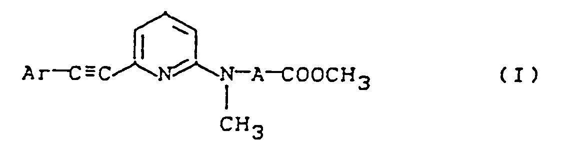 in welcher
- Ar: für jeweils gegebenenfalls einfach oder mehrfach, gleich oder verschieden substituiertes Phenyl oder Pyridyl steht, wobei als Substituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl und
- A: für einen Rest der Formel 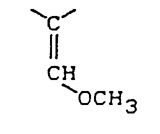 steht,
wobei die Verbindung 3-Methoxy-1-{N-[6-(4-chlorphenyl-ethinyl)-2-pyridyl]-N-methyl}-amino-acrylsäuremethylester, sowie die Verbindung 3-Methoxy-1-{N-[6-(phenyl-ethinyl)-2-pyridyl]-N-methyl}-amino-acrylsäuremethylester ausgenommen sind gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Pyridyl-Derivate der allgemeinen Formel (I), 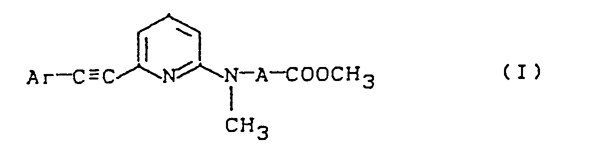 in welcher Ar und A die oben angegebenen Bedeutungen haben, erhält, wenn man
a) Phenylethinyl-Verbindungen der Formel (II),

   Ar-C≡CH (II)

   in welcher
   - Ar: die oben angegebene Bedeutung hat,
   mit N-(6-Brom-2-pyridyl)-sarcosinmethylester der Formel (III) 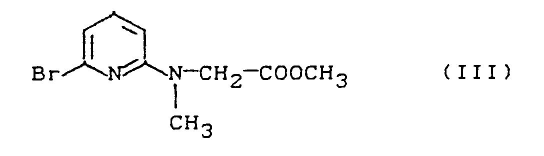 gegebenenfalls in Gegenwart eines Verdünnungsmittels, gegebenenfalls in Gegenwart eines Katalysators und gegebenenfalls in Gegenwart eines Reaktionshilfsmittels umsetzt oder wenn man
b) N-(6-Ethinyl-2-pyridyl)-sarcosinmethylester der Formel (IV), 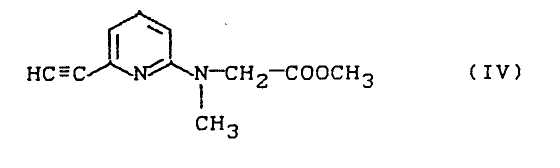 mit Halogenaromaten der Formel (V),

   Ar-X (V)

   in welcher
   - X: für Brom oder Iod steht und
   - Ar: die oben angegebene Bedeutung hat,
   gegebenenfalls in Gegenwart eines Verdünnungsmittels, gegebenenfalls in Gegenwart eines Katalysators und gegebenenfalls in Gegenwart eines Reaktionshilfsmittels umsetzt oder wenn man
c) die mit Hilfe der Verfahren (a) oder (b) erhältlichen Pyridyl-Derivate der Formel (Ia), 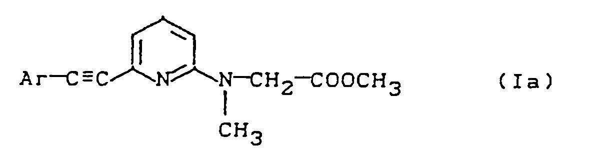 in welcher
   - Ar: die oben angegebene Bedeutung hat,
   in einer anschließenden Reaktionsfolge zunächst in einer ersten Stufe mit Orthoameisensäurediamidestern der Formel (VI), 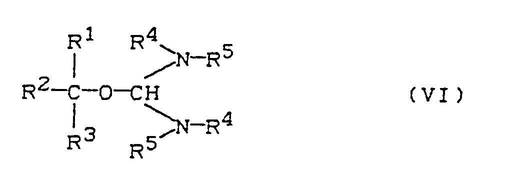 in welcher
   - R¹: für Wasserstoff oder Alkyl steht,
   - R² und R³: entweder unabhängig voneinander jeweils für Alkyl stehen oder gemeinsam mit dem Kohlenstoffatom, an welches sie gebunden sind, für einen Cycloalkylrest stehen und
   - R⁴ und R⁵: entweder unabhängig voneinander jeweils für Alkyl stehen oder gemeinsam mit dem Stickstoffatom, an welches sie gebunden sind, für einen Heterocyclus stehen, der gegebenenfalls weitere Heteroatome enthalten kann,
   gegebenenfalls in Gegenwart eines Verdünnungsmittels umsetzt und dann die so erhältlichen Aminoacrylsäureester der Formel (VII), 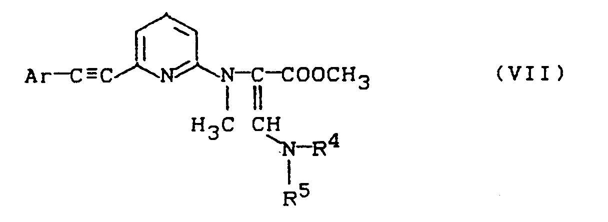 in welcher
   - Ar, R⁴ und R⁵: die oben angegebene Bedeutung haben,
   in einer anschließenden zweiten Stufe zunächst mit verdünnter Mineralsäure gegebenenfalls in Gegenwart eines Verdünnungsmittels hydrolysiert und anschließend die so erhältlichen Hydroxyacrylsäureester der Formel (VIII), 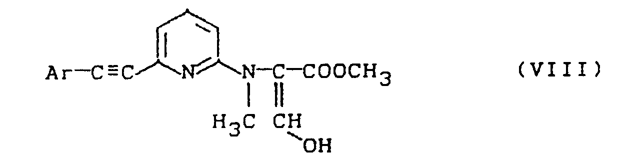 in welcher
   - Ar: die oben angegebene Bedeutung hat,
   in einer folgenden dritten Stufe mit Methylierungsmitteln gegebenenfalls in Gegenwart eines Verdünnungsmittels und gegebenenfalls in Gegenwart eines Reaktionshilfsmittels umsetzt.

Schließlich wurde gefunden, daß die neuen Pyridyl-Derivate der allgemeinen Formel (I) gute Wirksamkeit gegen Schädlinge besitzen.

Überraschenderweise zeigen die erfindungsgemäßen Verbindungen der allgemeinen Formel (I) eine bessere fungizide und akarizide Wirksamkeit als die aus dem Stand der Technik bekannten pyridylsubstituierten Acrylsäureester, welche chemisch und wirkungsmäßig naheliegende Verbindungen sind.

Die erfindungsgemäßen Pyridyl-Derivate sind durch die Formel (I) allgemein definiert. Bevorzugt sind Verbindungen der Formel (I), bei welchen
- Ar: für jeweils gegebenenfalls einfach bis dreifach, gleich oder verschieden substituiertes Phenyl, 2-Pyridyl, 3-Pyridyl oder 4-Pyridyl steht, wobei als Substituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen und
- A: für einen Rest der Formel 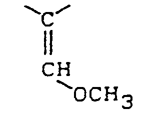 steht,
wobei die Verbindung 3-Methoxy-1-{N-[6-(4-chlorphenyl-ethinyl)-2-pyridyl]-N-methyl}-amino-acrylsäuremethylester, sowie die Verbindung 3-Methoxy-1-{N-[6-(phenyl-ethinyl)-2-pyridyl]-N-methyl}-amino-acrylsäuremethylester ausgenommen sind.

Ganz besonders bevorzugt sind Verbindungen der Formel (I), bei welchen
- Ar: für jeweils gegebenenfalls einfach oder zweifach, gleich oder verschieden substituiertes Phenyl, 2-Pyridyl oder 4-Pyridyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl oder Ethoximinoethyl und
- A: für einen Rest der Formel 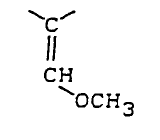 steht,
wobei die Verbindung 3-Methoxy-1-{N-[6-(4-chlorphenyl-ethinyl)-2-pyridyl]-N-methyl}-amino-acrylsäuremethylester, sowie die Verbindung 3-Methoxy-1-{N-[6-(phenyl-ethinyl)2-pyridyl]-N-methyl}-amino-acrylsäuremethylester ausgenommen sind.

Im einzelnen sei auf die bei den Herstellungsbeispielen genannten Verbindungen verwiesen.

Verwendet man beispielsweise N-(6-Brom-2-pyridyl)-sarcosinmethylester und 2-Ethinyl-5-methoxy-chlorbenzol als Ausgangsstoffe, so läßt sich der Reaktionsablauf des Verfahrens (a) durch das folgende Formelschema darstellen:

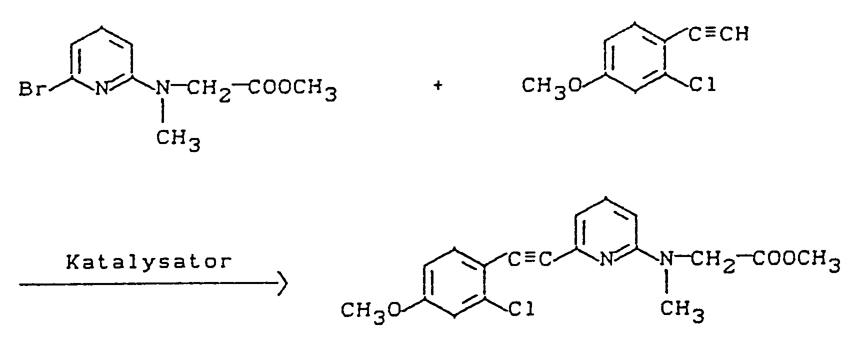

Verwendet man beispielsweise N-(6-Ethinyl-2-pyridyl)-sarcosinmethylester und 2,4-Dichlor-iodbenzol als Ausgangsstoffe, so läßt sich der Reaktionsablauf des Verfahrens (b) durch das folgende Formelschema darstellen:

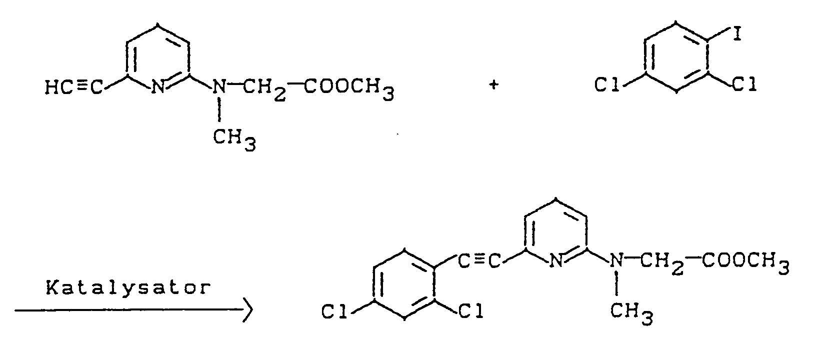

Verwendet man beispielsweise N-[6-(2-Chlor-4-fluor-phenylethinyl)-2-pyridinyl]-N-methyl-glycinmethylester und t-Butoxy-bis(dimethylamino)-methan als Ausgangsstoffe und Dimethylsulfat als Methylierungsmittel, so läßt sich der Reaktionsablauf des erfindungsgemäßen Verfahrens (c) durch das folgende Formelschema darstellen:

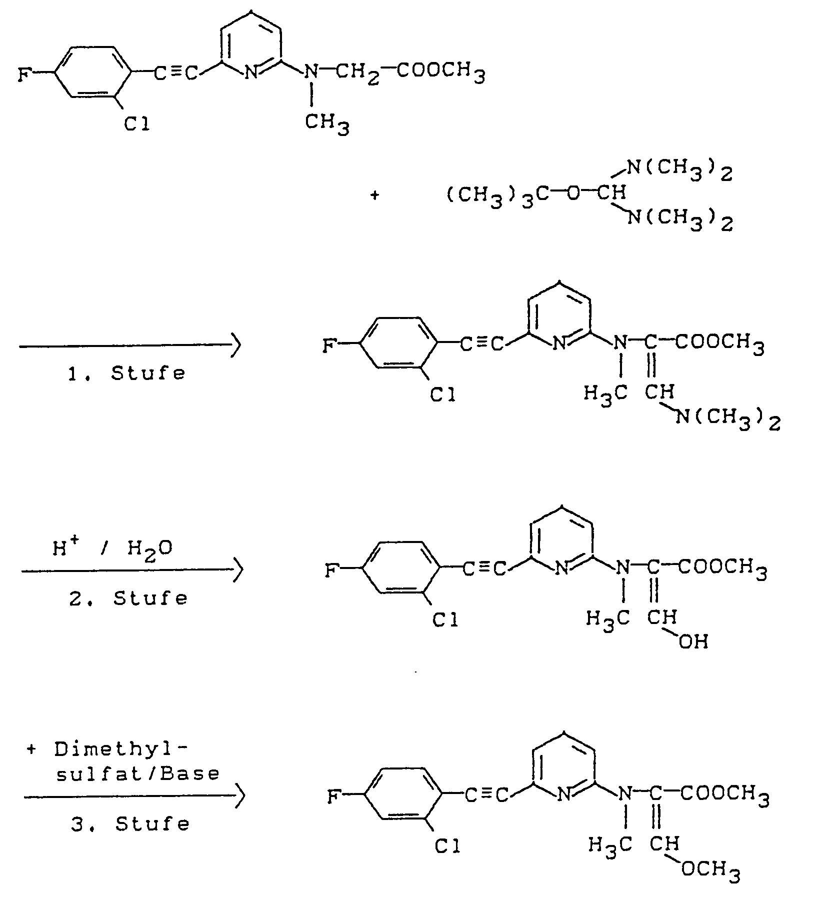

Die zur Durchführung des Verfahrens (a) als Ausgangsstoffe benötigten Phenylethinyl-Verbindungen sind durch die Formel (II) allgemein definiert. In dieser Formel (II) steht Ar vorzugsweise für diejenigen Reste, die bereits im Zusammenhang mit der Beschreibung der erfindungsgemäßen Verbindungen der Formel (I) als bevorzugt für diesen Substituenten genannt wurden.

Die Phenylethinyl-Verbindungen der Formel (II) sind bekannt oder erhältlich in Analogie zu bekannten Verfahren (vergleiche z. B. J. Chem. Soc., Perkin Trans. I, 1985, 2443; Zh. Org. Khim, 16, 1893 [1980]; Synthesis 1983, 312; Synthesis 1981, 364; J. Org. Chem. 46, 2280 [1981]).

Der zur Durchführung des Verfahrens (a) weiterhin als Ausgangsverbindung benötigte N-(6-Brom-2-pyridyl)-sarcosinmethylester der Formel (III) ist ebenfalls bekannt (vergleiche z. B. DE 39 04 931).

Der zur Durchführung des Verfahrens (b) als Ausgangsverbindung benötigte N-(6-Ethinyl-2-pyridyl)-sarcosinmethylester der Formel (IV) ist noch nicht bekannt.

Man erhält ihn, wenn man N-(6-Brom-2-pyridyl)-sarcosin-methylester der Formel (III) mit Trimethylsilylacetylen in Gegenwart eines Verdünnungsmittels, wie beispielsweise Triethylamin und in Gegenwart eines Katalysators, wie beispielsweise Bis-(triphenylphosphin)-palladium-(II)-chlorid/Kupfer-(I)-chlorid bei Temperaturen zwischen 20 und 150°C gegebenenfalls auch unter erhöhtem Druck in Analogie zur Durchführung der Verfahren (a) oder (b) umsetzt und anschließend die so erhältliche Verbindung N-[6-(2-Trimethylsilyl-ethinyl)-2-pyridyl]-sarcosinmethylester der Formel (IX) 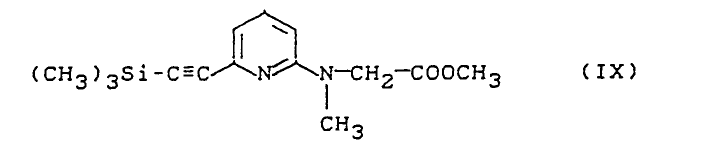 mit Basen, wie beispielsweise Kaliumcarbonat in Gegenwart eines Verdünnungsmittels, wie beispielsweise Methanol bei Temperaturen zwischen -20°C und +60°C hydrolysiert (vergleiche hierzu auch die Herstellungsbeispiele).
N-[6-(2-trimethylsilyl-ethinyl)-2-pyridyl]-sarcosinmethylester der Formel (IX) ist noch nicht bekannt.

Die zur Durchführung des erfindungsgemäßen Verfahrens (b) weiterhin als Ausgangsstoffe benötigten Halogenaromaten sind durch die Formel (V) allgemein definiert. In dieser Formel (V) steht Ar vorzugsweise für diejenigen Reste, die bereits im Zusammenhang mit der Beschreibung der erfindungsgemäßen Verbindungen der Formel (I) als bevorzugt für diesen Substituenten genannt wurden. X steht vorzugsweise für Brom oder Iod. Die Halogenaromaten der Formel (V) sind allgemein bekannte Verbindungen der organischen Chemie.

Die zur Durchführung des erfindungsgemäßen Verfahrens (c) als Ausgangsstoffe benötigten Pyridyl-Derivate sind durch die Formel (Ia) allgemein definiert. In dieser Formel (Ia) steht Ar vorzugsweise für diejenigen Reste, die bereits im Zusammenhang mit der Beschreibung der erfindungsgemäßen Verbindungen der Formel (I) als bevorzugt für diesen Substituenten genann wurden.

Die substituierten Pyridylpyrimidine der Formel (Ia) sind erhältlich mit Hilfe der Verfahren (a) oder (b).

Die zur Durchführung des erfindungsgemäßen Verfahrens (c) weiterhin als Ausgangsstoffe benötigten Orthoameisensäurediamidester sind durch die Formel (VI) allgemein definiert. In dieser Formel (VI) stehen R¹, R², R³, R⁴ und R⁵ vorzugsweise für geradkettiges oder verzweigtes Alkyl mit 1 bis 6 Kohlenstoffatomen, insbesondere für Methyl, Ethyl, n- oder i-Propyl sowie n-, i-, s- und t-Butyl.
Die Orthoameisensäurediamidester der Formel (VI) sind bekannt (vergleiche z. B. Chem. Ber. 101, 41-50 [1968]; Chem. Ber. 101, 1885-1888 [1968]; DE 23 03 919; PCT Int. Appl. WO 86/1204) oder erhältlich in Analogie zu bekannten Verfahren.

Die zur Durchführung des erfindungsgemäßen Verfahrens (c) weiterhin als Ausgangsstoffe benötigten Methylierungsmittel sind ebenfalls allgemein bekannte Verbindungen der organischen Chemie.

Als Verdünnungsmittel zur Durchführung der Verfahren (a) und (b) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder tertiäre Amine, wie Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Verfahren (a) und (b) können gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributylmethylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Verfahren (a) und (b) werden vorzugsweise in Gegenwart eines geeigneten Katalysators durchgeführt. Als solche kommen insbesondere Palladiumkatalysatoren, wie beispielsweise Palladiumchlorid, Palladiumacetat, Palladiumsulfat, elementares Palladium oder Palladium/Phosphin-Komplexe, wie beispielsweise Bis-(triphenylphosphin)-palladium-(II)-chlorid gegebenenfalls auf einem geeigneten Träger, wie beispielsweise Aktivkohle oder Siliciumdioxid auch in Gegenwart von Kupfer-(I)-Salzen, wie beispielsweise Kupferiodid, Kupferchlorid oder Kupferbromid infrage.

Die Reaktionstemperaturen können bei der Durchführung der erfindungsgemäßen Verfahren (a) und (b) in einem größeren Bereich variiert werden. Im allgemeinen arbeitet man bei Temperaturen zwischen 0°C und 150°C, vorzugsweise bei Temperaturen zwischen 50°C und 120°C.

Zur Durchführung des Verfahrens (a) setzt man pro Mol an Phenylethinyl-Verbindung der Formel (II) im allgemeinen 1.0 bis 2.0 Mol, vorzugsweise 1.0 bis 1.2 Mol an N-(6-Brom-2-pyridyl)-sarcosinmethylester der Formel (III) und 0.0001 bis 0.5 Mol, vorzugsweise 0.001 bis 0.1 Mol an Palladiumkatalysator sowie 0.0001 bis 0.5 Mol, vorzugsweise 0.001 bis 0.1 Mol an Kupfer-(I)-halogenid ein. Die Reaktionsdurchführung, Aufarbeitung und Isolierung der Reaktionsprodukte erfolgt in Analogie zu bekannten Verfahren (vergleiche hierzu beispielsweise Synthesis 1983, 312; Synthesis 1981, 364 oder die Herstellungsbeispiele).

Zur Durchführung des Verfahrens (b) setzt man pro Mol an N-(6-Ethinyl-2-pyridyl)-sarcosin-methylester der Formel (IV) im allgemeinen 1.0 bis 2.0 Mol, vorzugsweise 1.0 bis 1.2 Mol an Halogenaromat der Formel (V) und 0.0001 bis 0.5 Mol, vorzugsweise 0.001 bis 0.1 Mol an Katalysator sowie 0.001 bis 2.0 Mol, vorzugsweise 0.01 bis 1.0 Mol an Phasentransferkatalysator ein.
Die Reaktionsdurchführung, Aufarbeitung und Isolierung der Reaktionsprodukte erfolgt in Analogie zu bekannten Verfahren (vergleiche hierzu beispielsweise Synthesis 1983, 312; Synthesis 1981, 364 oder die Herstellungsbeispiele.

Als Verdünnungsmittel zur Durchführung der ersten Stufe des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Amide, wie N,N-Dimethylformamid, N,N-Dimethylacetamid, N-Methylformanilid, N-Methylpyrrolidon oder Hexamethylphosphorsäuretriamid.
Es ist auch möglich, die erste Stufe des erfindungsgemäßen Verfahrens (c) ganz ohne Zusatz von Lösungsmitteln durchzuführen.

Die Reaktionstemperaturen können bei der Durchführung der ersten Stufe des erfindungsgemäßen Verfahrens (c) in einem größeren Bereich variiert werden. Im allgemeinen arbeitet man bei Temperaturen zwischen -35°C und +150°C, vorzugsweise bei Temperaturen zwischen 0°C und 120°C.

Die erste Stufe des erfindungsgemäßen Verfahrens (c) kann auch unter vermindertem oder erhöhtem Druck durchgeführt werden, bevorzugt arbeitet man jedoch unter Normaldruck.

Gegebenenfalls kann es auch zweckmäßig sein, bei der ersten Stufe des erfindungsgemäßen Verfahrens (c) eine Inertgasatmosphäre, wie beispielsweise Stickstoff oder Argon zu verwenden. Im allgemeinen ist es jedoch möglich, das erfindungsgemäße Verfahren unter normaler Raumluftatmosphäre durchzuführen.

Zur Durchführung der ersten Stufe des erfindungsgemäßen Verfahrens (c) setzt man pro Mol an Pyridyl-Derivat der Formel (Ia) im allgemeinen 1.0 bis 15.0 Mol, vorzugsweise 1.0 bis 5.0 Mol an Orthoameisensäureester der Formel (VI) ein.
Die Reaktionsdurchführung, Aufarbeitung und Isolierung der Reaktionsprodukte erfolgt nach bekannten Verfahren (vergleiche hierzu beispielsweise DE 40 25 892 oder die Herstellungsbeispiele).

Als Säuren zur Durchführung der zweiten Stufe des erfindungsgemäßen Verfahrens (c) kommen übliche Mineralsäuren infrage. Vorzugsweise verwendet man verdünnte wässrige Salzsäure oder Schwefelsäure zur Hydrolyse der Enamine der Formel (VII).

Als Verdünnungsmittel zur Durchführung des erfindungsgemäßen Verfahrens (c) kommen polare organische Lösungsmittel oder wässrige Systeme infrage. Hierzu gehören insbesondere Ether, wie Dioxan, Tetrahydrofuran oder Ethylenglykoldimethyl- oder -diethylether; Ketone, wie Aceton; Nitrile, wie Acetonitril; Amide, wie N,N-Dimethylformamid, N,N-Dimethylacetamid, N-Methylformanilid, N-Methylpyrrolidon oder Hexamethylphosphorsäuretriamid oder Alkohole, wie Methanol, Ethanol, n- oder i-Propanol, deren Gemische mit Wasser oder reines Wasser.

Die Reaktionstemperaturen können bei der Durchführung der zweiten Stufe des erfindungsgemäßen Verfahrens (c) in einem größeren Bereich variiert werden. Im allgemeinen arbeitet man bei Temperaturen zwischen -20°C und 100°C, vorzugsweise bei Temperaturen zwischen 0°C und 60°C.

Zur Durchführung der zweiten Stufe des erfindungsgemäßen Verfahrens (c) setzt man pro Mol an Aminoacrylsäureester der Formel (VII) im allgemeinen 1.0 bis 5.0 Mol, vorzugsweise 2.0 bis 2.5 Mol an verdünnter wässriger Säure ein.
Die Reaktionsdurchführung, Aufarbeitung und Isolierung der Reaktionsprodukte erfolgt nach bekannten Verfahren (vergleiche hierzu beispielsweise DE 40 25 892 oder die Herstellungsbeispiele).

Als Methylierungsmittel zur Durchführung der dritten Stufe des erfindungsgemäßen Verfahrens (c) kommen alle üblichen Methylierungsmittel infrage. Beispielhaft genannt seien in diesem Zusammenhang Methyliodid, Dimethylsulfat oder Methyltosylat.

Als Verdünnungsmittel zur Durchführung der dritten Stufe des erfindungsgemäßen Verfahrens (c) kommen ebenfalls inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Die dritte Stufe des erfindungsgemäßen Verfahrens (c) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkylbenzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die dritte Stufe des erfindungsgemäßen Verfahrens (c) wird vorzugsweise in Gegenwart eines geeigneten Reaktionshilfsmittels durchgeführt. Als solche kommen alle üblichen anorganischen und organischen Basen infrage.

Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der dritten Stufe des erfindungsgemäßen Verfahrens (c) in einem größeren Bereich variiert werden. Im allgemeinen arbeitet man bei Temperaturen zwischen 0°C und 150°C, vorzugsweise bei Temperaturen zwischen 20°C und 100°C.

Zur Durchführung der dritten Stufe des erfindungsgemäßen Verfahrens (c) setzt man pro Mol an Hydroxyacrylsäureester der Formel (VIII) im allgemeinen 1.0 bis 10.0 Mol, vorzugsweise 1.0 bis 5.0 Mol an Methylierungsmittel und gegebenenfalls 1.0 bis 5.0 Mol, vorzugsweise 1.0 bis 2.5 Mol an Reaktionshilfsmittel ein.

Die Reaktionsdurchführung, Aufarbeitung und Isolierung der Reaktionsprodukte erfolgt nach bekannten Verfahren. In einer besonderen Durchführungsform ist es auch möglich, die zweite und die dritte Stufe des erfindungsgemäßen Verfahrens (c) in einem Reaktionsschritt ohne Isolierung der Zwischenprodukte der Formel (VIII) in einem sogenannten "Eintopfverfahren" durchzuführen (vergleiche auch die Herstellungsbeispiele).

Die Reinigung der Endprodukte der Formel (I) erfolgt mit Hilfe üblicher Verfahren, beispielsweise durch Säulenchromatographie oder durch Umkristallisieren.
Die Charakterisierung erfolgt mit Hilfe des Schmelzpunktes oder bei nicht kristallisierenden Verbindungen mit Hilfe der Protonen-Kernresonanzspektroskopie (¹H-NMR).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
   (Konidienform: Drechslera, Syn: Helminthosporium);
Cochliobolus-Arten, wie beispielsweise Cochliobolus sativus
   (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Getreidekrankheiten, wie beispielsweise gegen den Erreger der Netzfleckenkrankheit der Gerste (Pyrenophora teres) oder gegen den Erreger der Braunfleckenkrankheit an Gerste oder Weizen (Cochliobolus sativus) oder gegen den Erreger der Braunspelzigkeit des Weizens (Leptosphaeria nodorum) oder gegen den Erreger des echten Getreidemehltaues an Weizen oder Gerste (Erysiphe graminis) oder zur Bekämpfung von Krankheiten im Obst- und Gemüseanbau, wie beispielsweise gegen den Erreger des Apfelschorfes (Venturia inaequalis) oder gegen den Erreger des Mehltaues an Weinreben (Uncinula necator) oder zur Bekämpfung von Reiskrankheiten, wie beispielsweise gegen den Erreger der Reisfleckenkrankheit (Pyricularia oryzae) oder gegen den Erreger der Reisstengelkrankheit (Pellicularia sasakii) einsetzen. Daneben besitzen die erfindungsgemäßen Wirkstoffe eine breite in vitro-Wirksamkeit.

Auch die als Zwischenprodukt der Formeln (IV) und (IX) beschriebenen Verbindungen besitzen fungizide Wirksamkeit.

Darüberhinaus eignen sich die Wirkstoffe auch zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe akarizide Wirksamkeit aus.

Sie lassen sich mit besonders gutem Erfolg zur Bekämpfung von pflanzenschädigenden Milben, wie beispielsweise gegen die gemeine Spinnmilbe (Tetranychus urticae) einsetzen. Daneben besitzen die erfindungsgemäßen Wirkstoffe auch blattinsektizide Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln, Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z,B, Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B, gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalogcyaninfarbstoffe und Spurennährstoffe wie Salze von Eisen, Mangan, Bor, Kupfer, Kobalt, Molybdän und Zinn verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Fungizide in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können bei der Anwendung als Fungizide als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen bei der Anwendung als Fungizide in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen bei der Anwendung als Fungizide Wirkstoffmengen von 0,001 bis 50 g je Kilogramm Saatgut, vorzugsweise 0,01 bis 10 g benötigt.

Bei Behandlung des Bodens sind bei der Anwendung als Fungizide Wirkstoffkonzentrationen von 0,00001 bis 0,1 Gew.-%, vorzugsweise von 0,0001 bis 0,02 % am Wirkungsort erforderlich.

Die erfindungsgemäßen Wirkstoffe können bei der Anwendung als Insektizide und Akarizide in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner bei der Anwendung als Insektizide und Akarizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht bei der Anwendung als Insektizide und Akarizide in einer den Anwendungsformen angepaßten üblichen Weise.

Die Herstellung und die Verwendung der erfindungsgemäßen Wirkstoffe geht aus den nachfolgenden Beispielen hervor.

### Herstellungsbeispiele:

### Beispiel 1

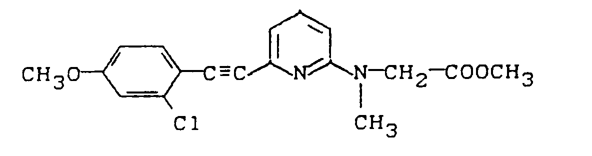

### (Verfahren a)

Eine Mischung aus 31,1 g (0,12 Mol) N-(6-Brom-2-pyridyl]-sarcosinmethylester, 20,0 g (0,12 Mol) 2-Ethinyl-5-methoxy-chlorbenzol (vergleiche z. B. J. Chem. Soc. Perkin Trans. I, 1985, 2443), 0,6 g (0,0085 Mol) Bis-(triphenylphosphin)-palladium-(II)-chlorid und 0,3 g (0,015 Mol) Kupfer-(I)-iodid in 300 ml Triethylamin wird 4 Stunden auf Rückflußtemperatur erhitzt, anschließend auf Raumtemperatur abgekühlt, filtriert, der Rückstand mit Dichlormethan nachgewaschen, das Filtrat im Vakuum eingeengt, der Rückstand in Essigester aufgenommen, mit Wasser gewaschen, über Natriumsulfat getrocknet, eingeengt und über Kieselgel (Laufmittel: Essigester / Hexan 1:1) chromatographiert.

Man erhält 25,1 g (61 % der Theorie) an N-{6-[2-(2-Chlor-4-methoxyphenyl)-ethinyl]-2-pyridyl}-sarcosinmethylester als Öl.
¹H-NMR (CDCl₃/Tetramethylsilan): δ = 3,73 (3H), 3,11 (3H), 4,43 (2H) ppm.

### Beispiel 2

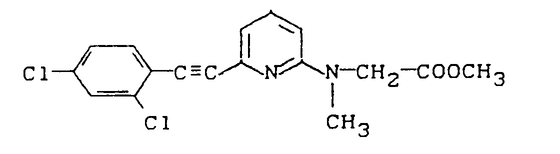

### (Verfahren b)

Zu 3,5 g (0,017 Mol) N-(6-Ethinyl-2-pyridyl)-sarcosinmethylester in 50 ml Triethylamin gibt man nacheinander 4,6 g (0,017 Mol) 1,3-Dichlor-4-iodbenzol, 0,105 g (0,00015 Mol) Bis-(triphenylphosphin)-palladium-(II)-chorid und 0,05 g (0,00026 Mol) Kupfer-(I)-iodid, rührt 16 Stunden bei 50°C, kühlt dann auf Raumtemperatur ab, nimmt die Mischung in Essigester auf, wäscht mit Wasser, trocknet über Natriumsulfat, engt im Vakuum ein und reinigt den Rückstand durch Chromatographie an Kieselgel (Laufmittel: Hexan/Essigester 3:1).

Man erhält 3,7 g (62 % der Theorie) an N-{6-[2-(2,4-Dichlorphenyl)-ethinyl]-2-pyridyl}-sarcosinmethylester als Öl.
¹H-NMR (CDCl₃/Tetramethylsilan): δ = 3,73 (3H), 3,12 (3H), 4,42 (2H) ppm.

### Herstellung der Ausgangsverbindung

### Beispiel IV:

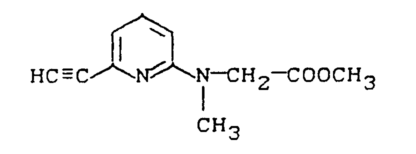

Zu 9,3 g (0,034 Mol) N-[6-(2-Trimethylsilyl-ethinyl)-2-pyridyl]-sarcosinmethylester in 200 ml Methanol gibt man 1,2 g (0,0087 Mol) Kaliumcarbonat und rührt anschließend 3 Stunden bei Raumtemperatur. Zur Aufarbeitung wird das Lösungsmittel im Vakuum abdestilliert, der Rückstand in Essigester aufgenommen, mit Wasser gewaschen, über Natriumsulfat getrocknet und im Vakuum eingeengt. Der Rückstand wird durch Chromatographie an Kieselgel (Laufmittel: Hexan / Essigester 1:1) gereinigt.

Man erhält 4,0 g (58 % der Theorie) an N-(6-Ethinyl-2-pyridyl)-sarcosinmethylester als Öl.
¹H-NMR (CDCl₃/Tetramethylsilan): δ = 3,72 (3H), 3,09 (3H), 4,40 (2H) ppm.

### Beispiel IX:

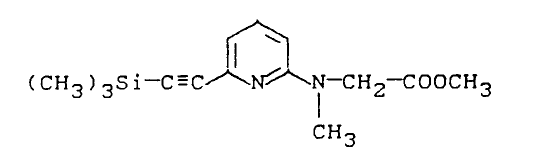

Zu 15,5 g (0,06 Mol) N-(6-Brom-2-pyridyl)-sarcosinmethylester und 9 g (0,09 Mol) Trimethylsilylacetylen in 60 ml Triethylamin gibt man 1,8 g (0,0026 Mol) Bis-(triphenylphosphin)-palladium-(II)-chlorid und 0,9 g (0,0047 Mol) Kupfer-(I)-iodid und erwärmt in einer verschlossenen Stahlapparatur unter dem sich einstellenden Eigendruck des Gemisches für 16 Stunden auf 90°C, kühlt dann auf Raumtemperatur ab, filtriert, wäscht den Rückstand mit Diethylether nach, wäscht das Filtrat dreimal mit Wasser, trocknet über Natriumsulfat, setzt Aktivkohle zu, filtriert abermals und engt im Vakuum ein. Der Rückstand wird durch Chromatographie an Kieselgel (Laufmittel: Hexan/Essigester 3:1) gereinigt.

Man erhält 9,3 g (56 % der Theorie) an N-[6-(2-trimethylsilyl-ethinyl)-2-pyridyl]-sarcosinmethylester vom Schmelzpunkt 85°C bis 86°C.

### Beispiel 3

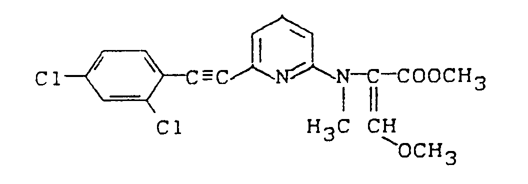

### (Verfahren c)

Zu 2,7 g (0,0066 Mol) 2-{N-Methyl-N-[6-(2,4-Dichlorphenyl)-ethinyl-2-pyridyl]-amino}-3-dimethylamino-acrylsäuremethylester in 110 ml Aceton und 110 ml Wasser gibt man 6,6 ml 2N Salzsäure und rührt anschließend 16 Stunden bei Raumtemperatur. Zur Aufarbeitung wird das Reaktionsgemisch mit 1,32 g (0,016 Mol) Natriumhydrogencarbonat versetzt, die organische Phase abgetrennt und im Vakuum eingeengt. Der Rückstand wird in 110 ml Dimethylformamid aufgenommen, mit 2,2 g (0,017 Mol) Dimethylsulfat und 1,85 g (0,013 Mol) Kaliumcarbonat versetzt und 16 Stunden bei Raumtemperatur gerührt. Zur Aufarbeitung gibt man Essigester und Wasser zu, trennt die organische Phase ab, wäscht noch dreimal mit Wasser nach, trocknet über Natriumsulfat und entfernt das Lösungsmittel im Vakuum. Der Rückstand wird über Kieselgel (Laufmittel: Hexan / Essigester 1:3) chromatographiert.

Man erhält 1,5 g (58 % der Theorie) an 2-{N-Methyl-N-[6-(2,4-Dichlorphenyl)-ethinyl-2-pyridyl]-amino}-3-methoxyacrylsäuremethylester als Öl.
¹H-NMR (CDCl₃/Tetramethylsilan): δ = 3,70 (3H), 3,24 (3H), 3,88 (3H) ppm.

### Herstellung der Ausgangsverbindung:

### Beispiel VII-1

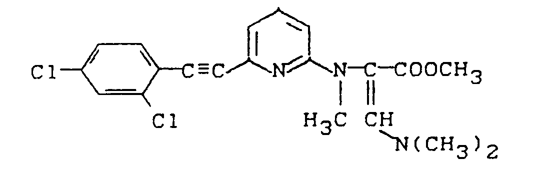

2,6 g (0,0074 Mol) N-{6-[2-(2,4-Dichlorphenyl)-ethinyl]-2-pyridyl}-sarcosinmethylester werden mit 3,24 g (0,019 Mol) t-Butoxy-bis-(dimethylamino)-methan versetzt und für drei Stunden auf 80°C erhitzt. Zur Aufarbeitung versetzt man die Reaktionsmischung mit Essigester und Wasser, trennt die organische Phase ab, trocknet über Natriumsulfat und entfernt das Lösungsmittel im Vakuum.

Man erhält 3,0 g (100 % der Theorie) an 2-{N-Methyl-N-[6-(2,4-Dichlorphenyl)-ethinyl-2-pyridyl]-amino}-3-dimethylamino-acrylsäuremethylester als Öl.
¹H-NMR (CDCl₃/Tetramethylsilan): δ = 3,61 (3H), 3,23 (3H), 2,95 (6H) ppm.

In entsprechender Weise und gemäß den allgemeinen Angaben zur Herstellung erhält man die folgenden pyridylsubstituierten Verbindungen der allgemeinen Formel (I):

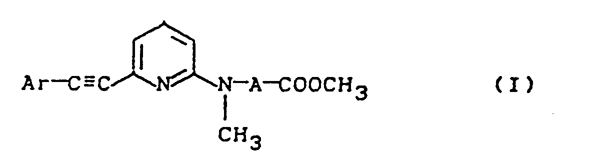

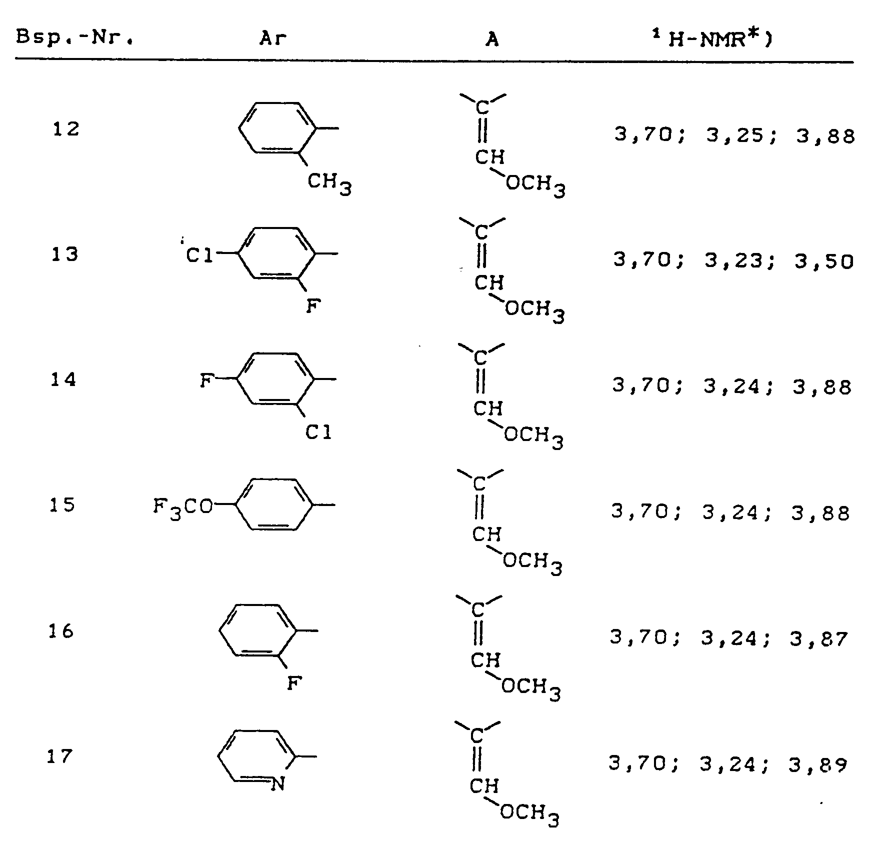

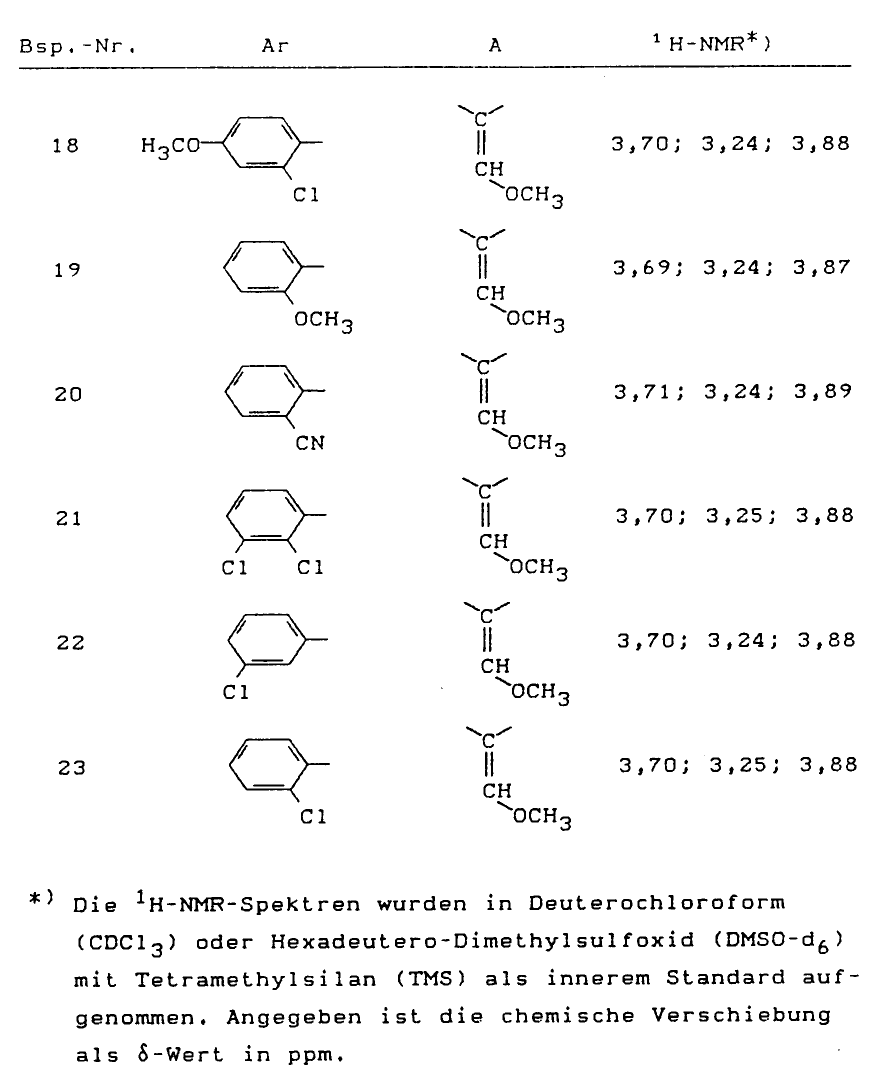

### Anwendungsbeispiele:

In den folgenden Anwendungsbeispielen wurden die nachstehend aufgeführten Verbindungen als Vergleichssubstanzen eingesetzt: 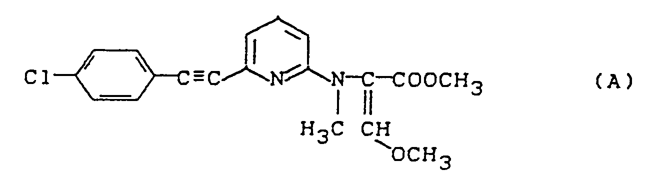 3-Methoxy-1-{N-[6-(4-chlorphenyl-ethinyl)-2-pyridyl]-N-methyl}-amino-acrylsäuremethylester (bekannt aus EP 383 117) 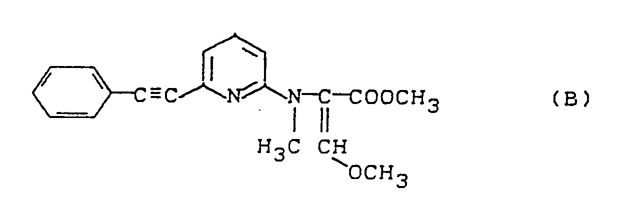 3-Methoxy-1-{N-[6-(phenyl-ethinyl)-2-pyridyl]-N-methyl}-amino-acrylsäuremethylester (bekannt aus EP 383 117)

### Beispiel A

### Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

In diesem Test zeigt die erfindungsgemäße Verbindung (13) bei einer Wirkstoffkonzentration von 5 ppm in der Spritzbrühe einen Wirkungsgrad von über 70%, während die Vergleichssubstanz (B) einen Wirkungsgrad von unter 10% aufweist.

### Beispiel B

### Pyrenophora teres-Test (Gerste) / protektiv

Lösungsmittel: 100 Gewichtsteile Dimethylformamid
Emulgator: 0,25 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

In diesem Test zeigen die in den Beispielen (16), (19) und (21) offenbarten Verbindungen bei einer Wirkstoffkonzentration von 250 ppm in der Spritzbrühe einen Wirkungsgrad von über 70%, während die Vergleichssubstanz (A) keine Wirksamkeit aufweist.

### Beispiel C

### Tetranychus Test (OP-resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe (Tetranychus urticae) befallen sind, werden mit einer Wirkstoffzubereitung der gewünschten Konzentration tropfnaß gespritzt.

Nach der gewünschten Zeit wird die Abtötung in Prozent bestimmt. Dabei bedeutet 100 %, daß alle Spinnmilben abgetötet wurden; 0 % bedeutet, daß keine Spinnmilben abgetötet wurden.

In diesem Test zeigt die in dem Beispiel (3) offenbarte Verbindung bei einer Wirkstoffkonzentration von 0,1% in der Spritzbrühe nach 13 Tagen einen Wirkungsgrad von 100%, während die Vergleichssubstanz (A) keine Wirksamkeit aufweist.

## Patentansprüche

1. Pyridyl-Derivate der Formel (I) 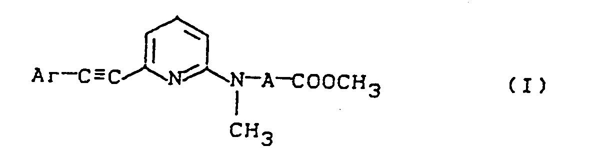 in welcher
Ar für jeweils gegebenenfalls einfach oder mehrfach, gleich oder verschieden substituiertes Phenyl oder Pyridyl steht, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl und
A für einen Rest der Formel 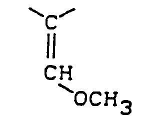 steht,
wobei die Verbindung 3-Methoxy-1-{N-[6-(4-chlor-phenyl-ethinyl)-2-pyridyl]-N-methyl}-amino-acrylsäuremethylester, sowie die Verbindung 3-Methoxy-1-{N-(6-phenyl-ethinyl)-2-pyridyl]-N-methyl}-amino-acrylsäuremethylester ausgenommen sind.

2. Verbindungen der Formel (I) gemäß Anspruch 1, bei welchen
Ar für jeweils gegebenenfalls einfach bis dreifach, gleich oder verschieden substituiertes Phenyl, 2-Pyridyl, 3-Pyridyl oder 4-Pyridyl steht, wobei als Substituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen und
A für einen Rest der Formel 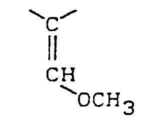 steht.

3. Verbindungen der Formel (I) gemäß Anspruch 1, bei welchen
Ar für jeweils gegebenenfalls einfach oder zweifach, gleich oder verschieden substituiertes Phenyl, 2-Pyridyl oder 4-Pyridyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl oder Ethoximinoethyl und
A für einen Rest der Formel 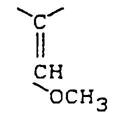 steht.

4. Verfahren zur Herstellung von Pyridyl-Derivaten der Formel (I) 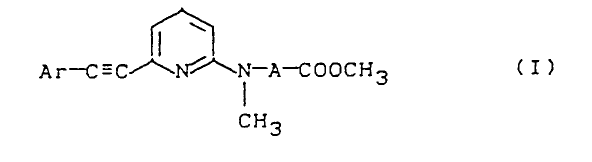 in welcher Ar und A die in Anspruch 1 angegebene Bedeutung haben, dadurch gekennzeichnet, daß man
a) Phenylethinyl-Verbindungen der Formel (II),
Ar-C≡CH (II)
in welcher
Ar die oben angegebene Bedeutung hat,
mit N-(6-Brom-2-pyridyl)-sarcosinmethylester der Formel (III) 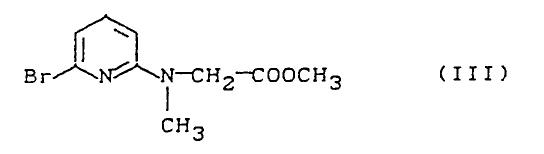 gegebenenfalls in Gegenwart eines Verdünnungsmittels, gegebenenfalls in Gegenwart eines Katalysators und gegebenenfalls in Gegenwart eines Reaktionshilfsmittels umsetzt oder
b) N-(6-Ethinyl-2-pyridyl)-sarcosinmethylester der Formel (IV), 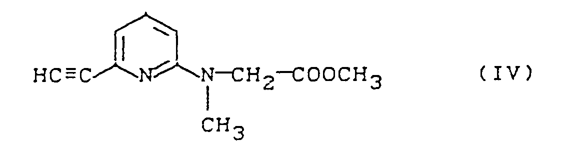 mit Halogenaromaten der Formel (V),
Ar-X (V)
in welcher
X für Brom oder Iod steht und
Ar die oben angegebene Bedeutung hat,
gegebenenfalls in Gegenwart eines Verdünnungsmittels, gegebenenfalls in Gegenwart eines Katalysators und gegebenenfalls in Gegenwart eines Reaktionshilfsmittels umsetzt oder
c) die mit Hilfe der Verfahren (a) oder (b) erhältlichen Pyridyl-Derivate der Formel (Ia), 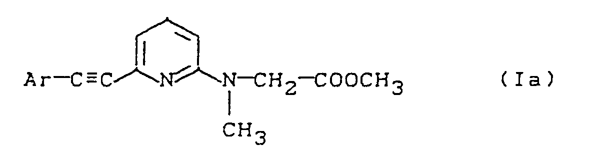 in welcher
Ar die oben angegebene Bedeutung hat,
in einer anschließenden Reaktionsfolge zunächst in einer ersten Stufe mit Orthoameisensäurediamidestern der Formel (VI), 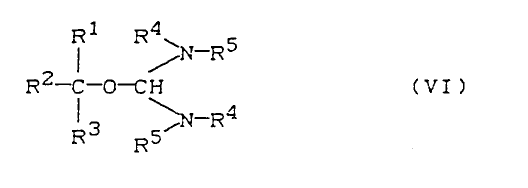 in welcher
R¹ für Wasserstoff oder Alkyl steht,
R² und R³ entweder unabhängig voneinander jeweils für Alkyl stehen oder gemeinsam mit dem Kohlenstoffatom, an welches sie gebunden sind, für einen Cycloalkylrest stehen und
R⁴ und R⁵ entweder unabhängig voneinander jeweils für Alkyl stehen oder gemeinsam mit dem Stickstoffatom, an welches sie gebunden sind, für einen Heterocyclus stehen, der gegebenenfalls weitere Heteroatome enthalten kann,
gegebenenfalls in Gegenwart eines Verdünnungsmittels umsetzt und dann die so erhältlichen Aminoacrylsäureester der Formel (VII), 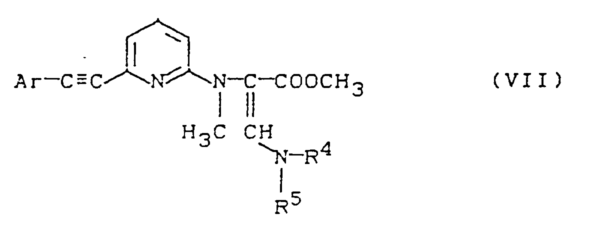 in welcher
Ar, R⁴ und R⁵ die oben angegebene Bedeutung haben,
in einer anschließenden zweiten Stufe zunächst mit verdünnter Mineralsäure gegebenenfalls in Gegenwart eines Verdünnungsmittels hydrolysiert und anschließend die so erhältlichen Hydroxyacrylsäureester der Formel (VIII), 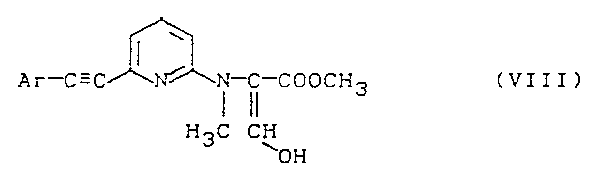 in welcher
Ar die oben angegebene Bedeutung hat,
in einer folgenden dritten Stufe mit Methylierungsmitteln gegebenenfalls in Gegenwart eines Verdünnungsmittels und gegebenenfalls in Gegenwart eines Reaktionshilfsmittels umsetzt.

5. Schädlingsbekampfungsmittel, gekennzeichnet durch einen Gehalt an mindestens einem Pyridyl-Derivat der Formeln (I) nach den Ansprüchen 1 bis 4.

6. Verwendung von Pyridyl-Derivaten der Formel (I), nach den Ansprüchen 1 bis 4 zur Bekämpfung von Schädlingen.

7. Verfahren zur Bekämpfung von Schädlingen, dadurch gekennzeichnet, daß man Pyridyl-Derivate der Formel (I) nach den Ansprüchen 1 bis 4 auf Schädlinge und/oder ihren Lebensraum einwirken läßt.

8. Verfahren zur Herstellung von Schädlingsbekämpfungsmitteln, dadurch gekennzeichnet, daß man Pyridyl-Derivate der Formel (I) nach den Ansprüchen 1 bis 4 mit Streckmitteln und/oder oberflächenaktiven Mitteln vermischt.

## Claims

1. Pyridyl derivatives of the formula (I) 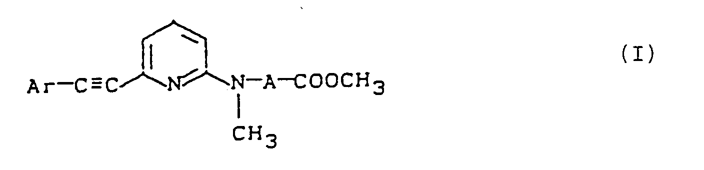 in which
Ar represents phenyl or pyridyl, each of which is optionally monosubstituted or polysubstituted by identical or different substituents, suitable substituents in each case being: halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, as well as phenyl which is optionally monosubstituted or polysubstituted by identical or different substituents from the series comprising halogen and/or straight-chain or branched alkyl having 1 to 4 carbon atoms, and
A represents a radical of the formula 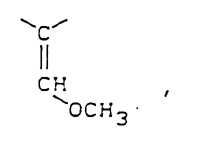
the compound methyl 3-methoxy-1-{N-[6-(4-chlorophenyl-ethinyl)-2-pyridyl]-N-methyl}-amino-acrylate, as well as the compound methyl 3-methoxy-1-{N-(6-phenyl-ethinyl)-2-pyridyl]-N-methyl}-amino-acrylate being excepted.

2. Compounds of the formula (I) according to Claim 1, in which
Ar represents phenyl, 2-pyridyl, 3-pyridyl or 4-pyridyl, each of which is optionally monosubstituted to trisubstituted by identical or different substituents, suitable substituents in each case being:
halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, and
A represents a radical of the formula

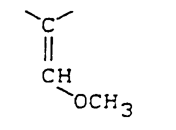

3. Compounds of the formula (I) according to Claim 1, in which
Ar represents phenyl, 2-pyridyl or 4-pyridyl, each of which is optionally monosubstituted or disubstituted by identical or different substituents, suitable substituents in each case being:
fluorine, chlorine, bromine, cyano, nitro, methyl, ethyl, n- or i-propyl, n-, i-, s- or -butyl, methoxy, ethoxy, n- or i-propoxy, n-, i-, s- or t-butoxy, methylthio, ethylthio, trifluoromethyl, trifluoromethoxy, trifluoromethylthio, methoxycarbonyl, ethoxycarbonyl, methoximinomethyl, methoximinoethyl, ethoximinomethyl or ethoximinoethyl, and
A represents a radical of the formula

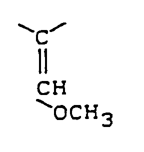

4. Process for the preparation of pyridyl derivatives of the formula (I) 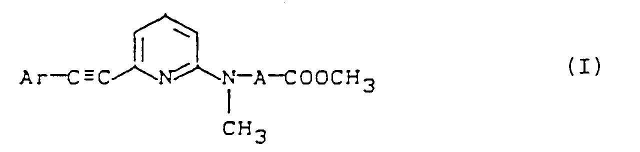 in which Ar and A have the meaning given in Claim 1, characterised in that
a) phenylethinyl compounds of the formula (II)
Ar-C≡CH (II)
in which
Ar has the abovementioned meaning,
are reacted with methyl N-(6-bromo-2-pyridyl)-sarcosinate, of the formula (III), 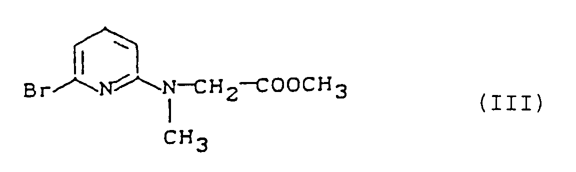 if appropriate in the presence of a diluent, if appropriate in the presence of a catalyst and if appropriate in the presence of a reaction auxiliary, or
b) methyl N-(6-ethinyl-2-pyridyl)-sarcosinate, of the formula (IV), 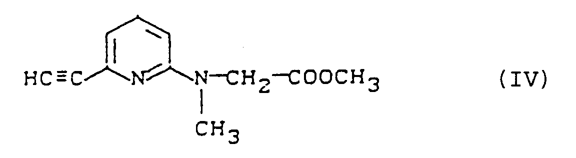 is reacted with halogenoaromatics of the formula (V)
Ar-X (V)
in which
X represents bromine or iodine and
Ar has the abovementioned meaning,
if appropriate in the presence of a diluent, if appropriate in the presence of a catalyst and if appropriate in the presence of a reaction auxiliary, or
c) the pyridyl derivatives of the formula (Ia) 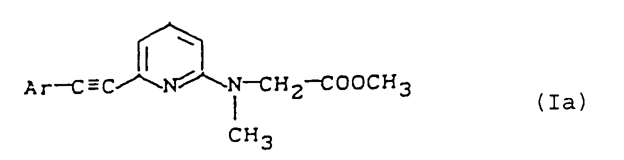 in which
Ar has the abovementioned meaning,
and which can be obtained with the aid of processes (a) or (b),
are reacted, in a subsequent reaction sequence, initially in a first step with orthoformic acid diamide esters of the formula (VI) 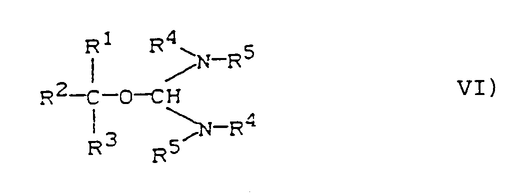 in which
R¹ represents hydrogen or alkyl,
R² and R³ either independently of one another in each case represent alkyl, or, together with the carbon atom to which they are bonded, represent a cycloalkyl radical, and
R⁴ and R⁵ either independently of one another in each case represent alkyl, or, together with the nitrogen atom to which they are bonded, represent a heterocycle which can optionally contain further hetero atoms,
if appropriate in the presence of a diluent, and the resulting aminoacrylates of the formula (VII) 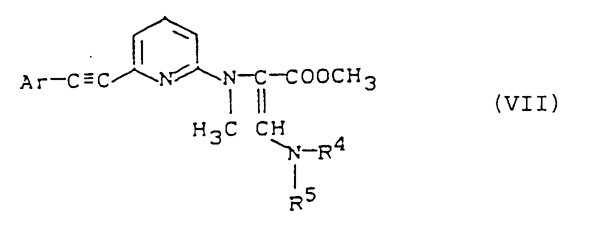 in which
Ar, R⁴ and R⁵ have the abovementioned meaning,
are then, in a subsequent second step, first hydrolysed with dilute mineral acid, if appropriate in the presence of a diluent, and the resulting hydroxyacrylates of the formula (VIII) 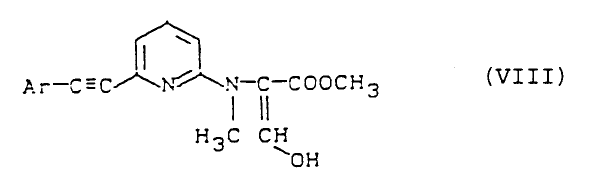 in which
Ar has the abovementioned meaning,
are then reacted, in a subsequent third step, with methylating agents, if appropriate in the presence of a diluent and if appropriate in the presence of a reaction auxiliary.

5. Pesticides, characterised in that they contain at least one pyridyl derivative of the formula (I) according to Claims 1 to 4.

6. Use of pyridyl derivatives of the formula (I) according to Claims 1 to 4 for combating pests.

7. Method of combating pests, characterised in that pyridyl derivatives of the formula (I) according to Claims 1 to 4 are allowed to act on pests and/or their environment.

8. Process for the preparation of pesticides, characterised in that pyridyl derivatives of the formula (I) according to Claims 1 to 4 are mixed with extenders and/or surface-active agents.

## Revendications

1. Dérivés de pyridyle de formule (I) 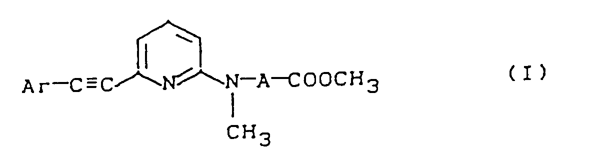 dans laquelle
Ar représente un groupe phényle ou un groupe pyridyle portant chacun le cas échéant un ou plusieurs substituants identiques ou différents, les substituants considérés dans chaque cas étant : un halogène, un groupe cyano, nitro, un groupe alkyle, un groupe alkoxy ou un groupe alkylthio ayant chacun 1 à 4 atomes de carbone et étant chacun linéaire ou ramifié, un groupe halogénalkyle, un groupe halogénalkoxy ou un groupe halogénalkylthio étant chacun linéaire ou ramifié et ayant chacun 1 à 4 atomes de carbone et 1 à 9 atomes d'halogènes identiques ou différents, un groupe alkoxycarbonyle ou un groupe alkoxyiminoalkyle étant chacun linéaire ou ramifié et ayant chacun 1 à 4 atomes de carbone dans les parties alkyle individuelles, ainsi qu'un groupe phényle portant le cas échéant un ou plusieurs substituants, identiques ou différents, halogéno et/ou alkyle linéaire ou ramifié ayant 1 à 4 atomes de carbone et
A représente un reste de formule 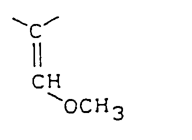 le composé ester méthylique d'acide 3-méthoxy-1-{N-[6-(4-chlorophényl-éthynyl)-2-pyridyl]-N-méthyl}-amino-acrylique ainsi que le composé ester méthylique d'acide 3-méthoxy-1-{N-(6-phényléthynyl)-2-pyridyl]-N-méthyl}-amino-acrylique étant exclus.

2. Composés de formule (I) suivant la revendication 1, dans lesquels
Ar représente un groupe phényle, un groupe 2-pyridyle, un groupe 3-pyridyle ou un groupe 4-pyridyle portant chacun le cas échéant un à trois substituants identiques ou différents, les substituants considérés dans chaque cas étant : un halogène, un groupe cyano, nitro, un groupe alkyle, un groupe alkoxy ou un groupe alkylthio ayant chacun 1 à 4 atomes de carbone et étant chacun linéaire ou ramifié, un groupe halogénalkyle, un groupe halogénalkoxy ou un groupe halogénalkylthio étant chacun linéaire ou ramifié et ayant chacun 1 à 4 atomes de carbone et 1 à 9 atomes d'halogènes identiques ou différents, un groupe alkoxycarbonyle ou un groupe alkoximino-alkyle étant chacun linéaire ou ramifié et ayant chacun 1 à 4 atomes de carbone dans les parties alkyle individuelles et
A représente un reste de formule

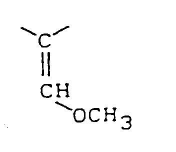

3. Composés de formule (I) suivant la revendication 1, dans lesquels
Ar représente un groupe phényle, un groupe 2-pyridyle ou un groupe 4-pyridyle portant chacun le cas échéant un ou deux substituants identiques ou différents, les substituants considérés dans chaque cas étant : le fluor, le chlore, le brome, un groupe cyano, nitro, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, trifluorométhyle, trifluorométhoxy, trifluoro-méthylthio, méthoxycarbonyle, éthoxycarbonyle, méthoximinométhyle, méthoximinoéthyle, éthoximinométhyle ou éthoximinoéthyle et
A représente un reste de formule

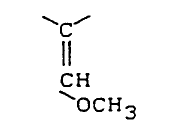

4. Procédé de production de dérivés de pyridyle de formule (I) 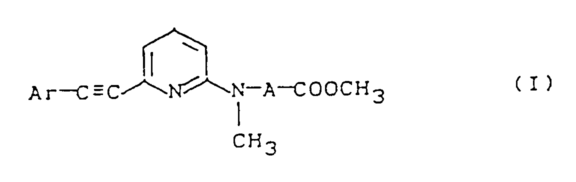 dans laquelle Ar et A ont la définition indiquée dans la revendication 1, caractérisé en ce que
a) on fait réagir des composés phényléthynyliques de formule (II),
Ar-C≡CH (II)
dans laquelle
Ar a la définition indiquée ci-dessus,
avec l'ester méthylique de N-(6-bromo-2-pyridyl)-sarcosine de formule (III) 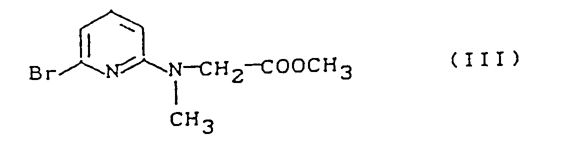 le cas échéant en présence d'un diluant, en la présence éventuelle d'un catalyseur et le cas échéant en présence d'un auxiliaire de réaction, ou bien
b) on fait réagir l'ester méthylique de N-(6-éthynyl-2-pyridyl)-sarcosine de formule (IV) 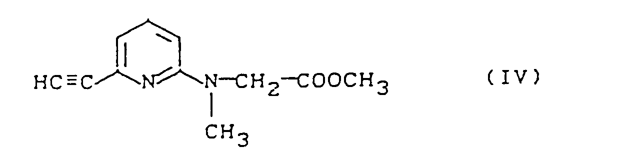 avec des hydrocarbures aromatiques halogénés de formule (V)
Ar-X (V)
dans laquelle
X représente le brome ou l'iode et
Ar a la définition indiquée ci-dessus,
le cas échéant en présence d'un diluant, en la présence éventuelle d'un catalyseur et, le cas échéant, en présence d'un auxiliaire de réaction, ou bien
c) on fait réagir les dérivés de pyridyle obtenus à l'aide des procédés (a) ou (b), de formule (Ia), 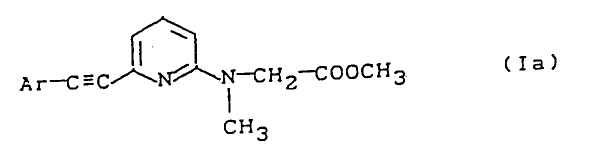 dans laquelle
Ar a la définition indiquée ci-dessus,
dans une succession de réactions, tout d'abord dans une première étape avec les esters de diamides d'acide orthoformique de formule (VI) 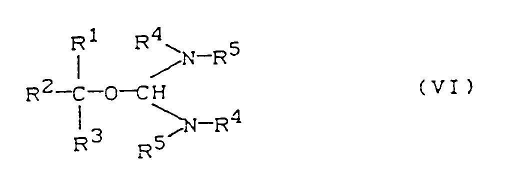 dans laquelle
R¹ représente de l'hydrogène ou un groupe alkyle,
R² et R³ représentent chacun, indépendamment l'un de l'autre, un groupe alkyle ou forment un reste cycloalkyle conjointement avec l'atome de carbone auquel ils sont liés et
R⁴ et R⁵ représentent chacun, indépendamment l'un de l'autre, un groupe alkyle ou forment, conjointement avec l'atome d'azote auquel ils sont liés, un hétérocycle qui peut contenir le cas échéant d'autres hétéroatomes,
éventuellement en présence d'un diluant puis on hydrolyse, éventuellement en présence d'un diluant, les esters d'acide aminoacrylique ainsi obtenus de formule (VII) 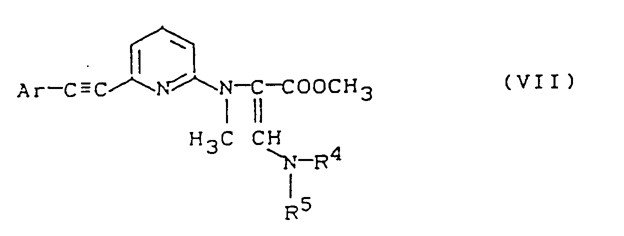 dans laquelle
Ar, R⁴ et R⁵ ont la définition indiquée ci-dessus,
dans une seconde étape subséquente, tout d'abord avec un acide minéral dilué puis on fait réagir les esters d'acides hydroxyalkyliques ainsi obtenus de formule (VIII) 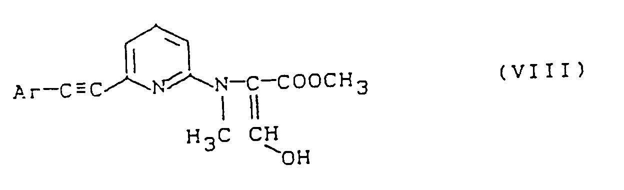 dans laquelle
Ar a la définition indiquée ci-dessus,
dans une troisième étape subséquente avec des agents de méthylation éventuellement en présence d'un diluant et en la présence éventuelle d'un auxiliaire de réaction.

5. Compositions pesticides, caractérisées par une teneur en au moins un dérivé de pyridyle de formule (I) suivant les revendications 1 à 4.

6. Utilisation de dérivés de pyridyle de formule (I) suivant les revendications 1 à 4 pour combattre des parasites.

7. Procédé pour combattre des parasites, caractérisé en ce qu'on fait agir des dérivés de pyridyle de formule (I) suivant les revendications 1 à 4 sur les parasites et/ou sur leur milieu.

8. Procédé de préparation de compositions pesticides, caractérisé en ce qu'on mélange des dérivés de pyridyle de formule (I) suivant les revendications 1 à 4 avec des diluants et/ou des agents tensio-actifs.
